# EUROPÄISCHE PATENTSCHRIFT

(11) **EP 2 922 889 B1**
(45) Veröffentlichungstag und Bekanntmachung des Hinweises auf die Patenterteilung: **10.05.2017**
(21) Anmeldenummer: 13794888.1
(22) Anmeldetag: 20.11.2013
(51) Int. Cl.: C08G 59/16, C08G 59/17, C08G 59/14

(54) **VERFAHREN ZUR HERSTELLUNG VON MODIFIZIERTEN EPOXY(METH)ACRYLATHARZEN UND IHRE VERWENDUNG**
METHOD FOR PRODUCING MODIFIED EPOXY(METH)ACRYLATE RESINS, AND THE USE THEREOF
PROCÉDÉ DE PRODUCTION DE RÉSINES ÉPOXY(MÉTH)ACRYLATE MODIFIÉES ET LEUR UTILISATION

(30) Priorität: 23.11.2012 DE 102012221441
(43) Veröffentlichungstag der Anmeldung: 30.09.2015
(73) Patentinhaber: Hilti Aktiengesellschaft, 9494 Schaan (LI)
(72) Erfinder: GAEFKE, Gerald, 86159 Augsburg (DE); BÜRGEL, Thomas, 86899 Landsberg am Lech (DE); LEITNER, Michael, 86899 Landsberg (DE)
(86) Internationale Anmeldenummer: PCT/EP2013/074234
(87) Internationale Veröffentlichungsnummer: WO 2014/079856

(56) Entgegenhaltungen:
- EP-A1- 0 589 831
- EP-A1- 0 632 079
- JP-A- 2002 275 237

## Beschreibung

Die Erfindung betrifft die Verwendung von modifizierten Epoxy(meth)acrylaten als radikalisch härtbare Bindemittel.

Nach dem Stand der Technik werden Epoxy(meth)acrylate durch regioselektive, ringöffnende nukleophile Addition von Acryl- oder Methacrylsäure an Epoxidgruppen aufweisende organische Verbindungen, z.B. Bisphenol-A-Diglycidylether, in Gegenwart von geeigneten basischen Katalysatoren, z.B. heteroaromatische Stickstoff-Verbindungen, erhalten (z.B. DE-P-4004091). Dabei werden ohne Nachbehandlung direkt einsetzbare und durch Radikalinitiatoren polymerisierbare Monomere erhalten.

Nachteilig an Bindemitteln auf Basis dieser Monomere für die Anwendung in Mörtelmassen für die chemische Befestigung sind deren relativ geringe Verbundtraglasten, die geringe dreidimensionale Vernetzung, der starke Schrumpf sowie die Inhibierung der Oberflächenaushärtung durch Sauerstoff. Ferner ist der hohe Restdoppelbindungsgehalt nach der vernetzenden Polymerisation nachteilig, wodurch keine hohen Verbundtraglasten erreicht werden können.

Dementsprechend werden bei chemischen Befestigungssystemen mit einem Bindemittel auf der Basis von Epoxy(meth)acrylaten weitere Verbindungen zugesetzt, welche den genannten Nachteilen entgegenwirken. Um hohe Verbundtraglasten sowie eine gute dreidimensionale Vernetzung zu erreichen, werden co-polymerisierbare Monomere eingesetzt, die entweder zwei radikalisch polymerisierbare Endgruppen oder eine radikalisch polymerisierbare Endgruppe und eine polare Endgruppe, wie eine Hydroxylgruppe, aufweisen. Häufig werden hierzu Hydroxyalkyl(meth)acrylate eingesetzt. Diese haben jedoch den Nachteil, dass einige Vertreter gesundheitsgefährdend und daher kennzeichnungspflichtig sind, wie das häufig verwendete Hydroxypropylmethacrylat (HPMA), das als reizend gekennzeichnet werden muss. Dies führt dazu, dass Reaktionsharz-Systeme, die diese Verbindungen in einer bestimmten Menge enthalten, ebenfalls kennzeichnungspflichtig werden.

Ein anderer Ansatz hatte die Reduzierung der freien Hydroxylgruppen und damit die Reduzierung der hydrophilen Gruppen zum Gegenstand. Zur Modifizierung des Additionsproduktes von Bisphenol-A-diglycidylether und Methacrylsäure, genannt Bis-GMA, wurden dessen Hydroxylgruppen mit Methacrylsäure (US 43 57 456), Methacrylsäurechlorid (US 37 21 644), Lactonen (DE 33 34 329), Bernsteinsäureanhydrid (DE-P-2610146) oder mit Diisocyanaten (US 36 29 187) umgesetzt. Für diese Reaktionen werden jedoch lange Reaktionszeiten und relativ hohe Temperaturen benötigt, wodurch die Gefahr einer vorzeitigen Polymerisation des Bis-GMA während der Modifizierung extrem hoch ist. Da bekanntlich schon die Netzwerkpolymere des unmodifizierten Bis-GMA relativ hohe Restdoppelbindungsgehalte aufweisen, sind weitere Doppelbindungen im Molekül, wie in US 43 57 456 und US 37 21 644 vorgeschlagen, eher nachteilig.

Aus der EP 0 632 079 A1 sind höhermolekulare Epoxyacrylate sowie neue höhermolekulare carboxylgruppenhaltige Epoxyacrylate, Verfahren zu deren Herstellung, die Verwendung dieser Acrylate in Photoresistformulierungen und die Anwendung dieser Formulierungen vor allem auf dem Gebiet der Leiterplatten und der Druckplatten bekannt. Aus der JP 2002 275237 A ist ferner eine strahlungshärtende Verbindung aus der Reaktion zwischen einem Epoxy(meth)acrylat und einem Carbonsäureanhydrid zur Beschichtung von elektronischen Bauteilen, insbesondere als Löstopplack, bekannt. Eine Verwendung für chemische Befestigungssysteme wird in keinem der Dokumente erwähnt. Bei der Reaktion von Bis-GMA mit Lactonen bzw. des Dibenzoats von Bisphenol-A-diglycidylether mit Glycidyl(meth)acrylat werden wieder Hydroxylgruppen gebildet, d.h. die Hydrophilie des Moleküls wird unverändert bleiben.

Nachteilig an der Modifizierung ist ferner, dass gemäß der DE 4109048 A1 durch Reaktion der beiden Hydroxylgruppen des Bis-GMA mit bifunktionellen Verbindungen, wie Bernsteinsäureanhydrid, Polyaddukte entstehen, was zu stark erhöhter Viskosität der modifizierten Epoxy(meth)acrylate und damit zu einer erschwerten Weiterverarbeitung führen wird.

Es besteht daher Bedarf an einem einfachen Verfahren, bei dem keine Reinigung oder Abtrennung des Endprodukts erforderlich ist, so dass der Reaktionsansatz direkt eingesetzt werden kann, und bei dem keine vorzeitige Polymerisation der Edukte oder Produkte erfolgt.

Aufgabe der Erfindung ist die Bereitstellung eines Verfahrens zur Herstellung modifizierter Additionsprodukte von Acryl- oder Methacrylsäure an Epoxidgruppen aufweisende organische Verbindungen, das einfach durchzuführen und zu kontrollieren ist.

Erfindungsgemäß wird die Aufgabe durch die Verwendung eines Epoxy(meth)acrylatharzes als Bindemittel in radikalisch härtbaren Harzmischungen und diese Harzmischung enthaltende Reaktionsharzmörtel-Zusammensetzungen, insbesondere zur chemischen Befestigung gelöst, wobei das Epoxy(meth)acrylatharz durch ein Verfahren zur Herstellung von modifizierten Epoxy(meth)acrylaten bereitgestellt wird, bei dem Epoxidgruppen aufweisende organische Verbindungen mit (Meth)acrylsäure umgesetzt werden und nachdem mindestens 80% der Epoxidgruppen umgesetzt wurden, das Produkt mit dem Anhydrid einer gesättigten Dicarbonsäure umgesetzt wird. Dementsprechend erfolgt die Herstellung der modifizierten Epoxy(meth)acrylate in einer Zweistufen-Eintopfreaktion, aus der ein für alle Anwendungsgebiete direkt einsetzbares und lagerstabiles Produkt resultiert.

Zum besseren Verständnis der Erfindung werden die folgenden Erläuterungen der hierin verwendeten Terminologie als sinnvoll erachtet. Im Sinne der Erfindung bedeutet:
- "*Basisharz*" die reine, härtende bzw. härtbare Verbindung, die für sich oder mit Reaktionsmitteln, wie Härter, Beschleuniger und dergleichen (nicht im Basisharz enthalten), durch Polymerisation härtet; die härtbaren Verbindungen können Monomere, Dimere, Oligomere und Präpolymere sein;
- "*Harz-Masterbatch*" das Produkt der Herstellung des Basisharzes nach der Synthese (ohne Isolierung des Basisharzes) welches Reaktivverdünner, Stabilisatoren und Katalysatoren enthalten kann;
- "*Harzmischung*" eine Mischung aus dem Harz-Masterbatch und Beschleunigern sowie Stabilisatoren und ggf. weiteren Reaktivverdünnern; dieser Begriff wird gleichbedeutend mit dem Begriff "*organisches Bindemittel*" verwendet;
- "*Reaktionsharzmörtel*" eine Mischung aus Harzmischung und anorganischen Zuschlagstoffen; hierfür wird gleichbedeutend der Begriff "A Komponente" verwendet;
- "*Härtungsmittel*" Stoffe, welche die Polymerisation (das Härten) des Basisharzes bewirken;
- "*Härter*" eine Mischung aus Härtungsmittel und organischen und/oder anorganischen Zuschlagstoffen;
- "*Beschleuniger*" eine zur Beschleunigung der Polymerisationsreaktion (Härtung) fähige Verbindung, die dazu dient, die Bildung des Radikalstarters zu beschleunigen;
- "*Polymerisationsinhibitor*" eine zur Inhibierung der Polymerisationsreaktion (Härtung) fähige Verbindung, die einmal dazu dient, die Polymerisationsreaktion und damit ein unerwünschtes vorzeitiges Polymerisieren der radikalisch polymerisierbaren Verbindung während der Lagerung zu vermeiden, wobei diese Verbindungen üblicherweise in so geringen Mengen eingesetzt werden, dass die Gelzeit nicht beeinflusst wird; andererseits dient der Polymerisationsinhibitor dazu, die Polymerisationsreaktion unmittelbar nach der Zugabe des Härtungsmittels zu verzögern, wobei die Verbindungen üblicherweise in solchen Mengen eingesetzt werden, dass die Gelzeit beeinflusst wird;
- "*Reaktivverdünner*" flüssige oder niedrigviskose Basisharze, welche andere Basisharze, den Harz-Masterbatch oder die Harzmischung verdünnen und dadurch die zu deren Applikation notwendige Viskosität verleihen, zur Reaktion mit dem Basisharz befähigte funktionelle Gruppen enthalten und bei der Polymerisation (Härtung) zum überwiegenden Teil Bestandteil der gehärteten Masse (Mörtel) werden; auch co-polymerisierbares Monomer genannt.
- "*Gelzeit*" für ungesättigte Polyester- oder Vinyl-Harze, die gewöhnlich mit Peroxiden gehärtet werden, entspricht die Zeit der Härtungsphase des Harzes der Gelzeit, in der sich die Temperatur des Harzes von +25°C auf +35°C erhöht. Dies entspricht in etwa dem Zeitraum, in dem sich die Fluidität oder Viskosität des Harzes noch in so einem Bereich befindet, dass das Reaktionsharz bzw. die Reaktionsharzmasse noch einfach ver- bzw. bearbeitet werden kann;
- "*Mörtelmasse*" bezeichnet eine Formulierung, die neben der Reaktionsharzmasse weitere organische oder anorganische Füllstoffe enthält und die als solches direkt zur chemischen Befestigung verwendet werden kann;
- "*(Meth)acryl...*/*...(meth)acryl...*", dass sowohl die "Methacryl.../...methacryl..."- als auch die "Acryl.../...acryl..."-Verbindungen umfasst sein sollen;
- "*Epoxy(meth)acrylate*" Acrylat- oder Methacrylatgruppen aufweisende, im wesentlichen Epoxygruppen-freie Derivate von Epoxidharzen;
- "*Epoxidäquivalentmasse*" diejenige Menge an Epoxidharz in [g], die ein Äquivalent [Val] Epoxidfunktionen besitzt und berechnet sich aus der Molmasse M in [g/Mol] dividiert durch die Funktionalität *f* in [Val/Mol]; (EEW [g/Val]);
- "*Carboxyäquivalentmasse*" diejenige Menge an Carboxyverbindung in [g], die ein Äquivalent [Val] Carboxyfunktion besitzt und berechnet sich aus der Molmasse M in [g/Mol] dividiert durch die Funktionalität *f* in [Val/mol]; (COOH-EW [g/Val]);
- "*kalthärtend*", dass die Harzmischungen und Reaktionsharzmörtel bei Raumtemperatur vollständig aushärten können.

Zweckmäßig werden als Epoxidgruppen aufweisende organische Verbindungen solche mit einem Molekulargewicht eingesetzt, das einem Zahlenmittel der Molmasse *M̅ₙ* im Bereich von 129 bis 2.400 g/Mol entspricht und die im Mittel mindestens eine, bevorzugt 1,5 bis 2 Epoxidgruppen pro Molekül enthalten. Besonders bevorzugt sind die Epoxidgruppen vom Glycidylether- oder Glycidylestertyp, die durch Umsetzen eines Epihalohydrins, insbesondere Epichlorhydrin, mit einer mono- oder multifunktionellen aliphatischen oder aromatischen Hydroxyverbindungen, Thiolverbindungen oder Carbonsäuren oder einem Gemisch davon erhalten werden. Die erhaltenen Epoxidgruppen aufweisenden organischen Verbindungen weisen eine Epoxidäquivalentmasse (EEW) bevorzugt im Bereich von 87 bis 1.600 g/Val, stärker bevorzugt im Bereich von 160 bis 800 g/Val und am stärksten bevorzugt im Bereich von 300 bis 600 g/Val auf.

Beispiele geeigneter Epoxidgruppen aufweisender Verbindungen, jedoch nicht einschränkend, sind Polyglycidylether mehrwertiger Phenole wie beispielsweise Brenzkatechin, Resorcin, Hydrochinon, 4,4'-Dihydroxydiphenylmethan, 2,2-(4,4'-Dihydroxydiphenyl)propan (Bisphenol-A), Bis(4-hydroxyphenyl)methan (Bisphenol F), 4,4'-Dihydroxydiphenylsulfon (Bisphenol S), 4,4'-Dihydroxydiphenylcyclohexan, Tris-(4-hydroxyphenyl)-methan, Novolaken (d.h. aus Umsetzungsprodukten von ein- oder mehrwertigen Phenolen mit Aldehyden, insbesondere Formaldehyd, in Gegenwart saurer Katalysatoren), wie Phenol-Novolak-Harz, Cresol-Novolak-Harz.

Weiter können folgende beispielhaft, jedoch nicht einschränkend, genannt werden: Glycidylether von Monoalkoholen, wie beispielsweise n-Butanol oder 2-Ethylhexanol; oder Glycidylether mehrwertiger Alkohole, wie beispielsweise aus 1,4-Butandiol, 1,4-Butendiol, 1,6-Hexandiol, Glycerin, Benzylalkohol, Neopentylglykol, Ethylenglykol, Cyclohexandimethanol, Trimethylolpropan, Pentaerythrit und Polyethylenglykolen, Triglycidylisocyanurat; Polyglycidylthioether mehrwertiger Thiole, wie Bismercaptomethylbenzol; oder Glycidylester von Monocarbonsäuren, wie Versaticsäure; oder Glycidylester mehrwertiger, aromatischer und aliphatischer Carbonsäuren, beispielsweise Phthalsäurediglycidylester, Isophthalsäurediglycidylester, Terephthalsäurediglycidylester, Tetrahydrophthalsäurediglycidylester, Adipinsäurediglycidylester und Hexahydrophthalsäurediglycidylester.

Als Epoxidgruppen aufweisende organische Verbindungen sind Diglycidylether zweiwertiger Hydroxyverbindungen besonders bevorzugt, welche die allgemeinen Formel (I) aufweisen 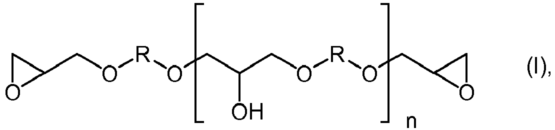 in der R eine unsubstituierte oder substituierte aliphatische oder aromatische Gruppe, bevorzugt eine aromatische Gruppe, stärker bevorzugt eine aromatische Gruppe mit 6 bis 24 Kohlenstoffatomen ist und der Mittelwert für n gleich 0 bis 3 ist. R ist besonders bevorzugt eine Gruppe vom Bisphenoltyp, wie Bisphenol A, Bisphenol F oder Bisphenol S, oder vom Novolaktyp, wobei eine Gruppe vom Bisphenoltyp ganz besonders bevorzugt ist. n ist bevorzugt etwa 0,1, etwa 1 oder etwa 2. Dabei werden im Sinne der vorliegenden Erfindung Verbindungen, in denen n ∼ 0,1 ist, als monomer und Verbindungen, in denen n ∼ 1 oder 2 ist, als polymer bezeichnet.

Die Epoxy(meth)acrylatharze werden in einem ersten Schritt (i) durch Umsetzung einer Epoxidgruppen aufweisenden organischen Verbindung mit Acrylsäure oder Methacrylsäure erhalten, so dass die Harze notwendigerweise Acryloxygruppen oder Methacryloxygruppen am Ende des Moleküls und Hydroxylgruppen in 2-Position zur gebildeten Acryloxy- bzw. Methacryloxygruppe (im Folgenden auch β-Hydroxylgruppen genannt) in der Hauptkette des Moleküls aufweisen. Zweckmäßig werden pro Epoxidäquivalent 0,7 bis 1,2 Carboxyläquivalente (Meth)acrylsäure eingesetzt. Die Epoxidgruppen aufweisenden organischen Verbindungen und die (Meth)acrylsäure werden dabei bevorzugt in etwa stöchiometrischen Verhältnissen eingesetzt, d.h. pro Epoxidäquivalent der organischen Verbindung wird etwa ein Äquivalent (Meth)acrylsäure eingesetzt.

Die Umsetzung der Epoxidgruppen aufweisenden organischen Verbindung mit der (Meth)acrylsäure erfolgt in an sich bekannter Weise durch Zusammenfügen der Komponenten.

Die Umsetzung erfolgt in Substanz oder in geeigneten Lösungsmitteln. Geeignete Lösungsmittel sind beispielsweise inerte Lösungsmittel, wie Butylacetat, Toluol, Cyclohexan oder Gemische derartiger Lösungsmittel, Monomere vom Glycidylester- oder Glycidylethertyp (Reaktivverdünner vom Typ I) oder copolymerisierbare Monomere (Reaktivverdünner vom Typ II), die nachstehend beispielhaft genannt werden. Vorzugsweise werden keine Lösungsmittel oder, falls erforderlich, Reaktivverdünner vom Typ I oder vom Typ II eingesetzt, wobei Reaktivverdünner vom Typ II stärker bevorzugt sind. Die inerten Lösungsmittel wirken in dem ausgehärteten Harz bzw. Zusammensetzung als Weichmacher, so dass deren Verwendung stark von der Verwendung der ausgehärteten Masse bzw. der Zusammensetzung abhängt.

Geeignete Reaktivverdünner vom Typ I sind Allylglycidylether, Butylglycidylether (BGE), 2-Ethylhexylglycidylether, Alkylglycidylether (C₁₂-C₁₄), Tridecylglycidylether, Phenylglycidylether (PGE), o-Kresolglycidylether (CGE), *p-tert*-Butylglycidylether, Resorcindiglycidylether (RDGE), 1,4-Butandioldiglycidylether (BDGE), 1,6-Hexandioldiglycidylether (HDGE), Cyclohexandimethanoldiglycidylether, Neopentylglycoldiglycidylether, Trimethylolpropantriglycidylether, Glycerintriglycidylether, Polypropylenglycoldiglycidylether sowie epoxidierte Pflanzenöle wie beispielsweise epoxidiertes Leinöl und epoxidiertes Rhizinusöl.

Die Epoxidgruppen aufweisende organische Verbindung, die (Meth)acrylsäure und gegebenenfalls das Lösungsmittel werden bei Raumtemperatur, insbesondere bei 10°C bis 40°C, im Reaktionskessel, vorzugsweise unter Rühren, vorgelegt und vermischt.

Die Umsetzung erfolgt dann bei etwa +80°C bis +120°C. Bevorzugt, insbesondere bei Epoxidgruppen aufweisenden Verbindung mit hohem Molekulargewicht, wird das Gemisch so schnell wie möglich auf diese Temperatur erhitzt, um ein Kleben des Gemisches an dem Reaktionskessel oder dem Rührer, zu verhindern. Bevorzugt erfolgt das Erhitzen über einen Zeitraum von 30 Minuten bis 3 Stunden, abhängig von der vorliegenden Menge, wobei je nach Molekulargewicht der Epoxidgruppen aufweisenden Verbindung der Zeitraum deutlich von diesem Zeitraum abweichen kann. Bei höheren Molekulargewichten ist ein kürzerer Zeitraum günstig und bei niedrigeren Molekulargewichten ist ein länger Zeitraum möglich.

Die Umsetzung der Epoxidgruppen aufweisenden Verbindung mit der (Meth)acrylsäure wird in Gegenwart von etwa 0,01 bis 3 Gew.-%, bezogen auf die Epoxidgruppen aufweisende Verbindung, an geeigneten Katalysatoren durchgeführt. Geeignete Katalysatoren sind etwa tertiäre Amine, quartäre Ammoniumsalze, Alkalihydroxide, Alkalisalze organischer Carbonsäuren, Mercaptane, Dialkylsulfide, Sulfoniumverbindungen, Phosphoniumverbindungen oder Phosphine. Bevorzugt werden quartäre Ammoniumsalze, wie Tetraethylammoniumchlorid, Tetraethylammoniumbromid, Tetrabutylammoniumchlorid, Tetrabutylammoniumbromid und dergleichen, verwendet.

Der Katalysator wird nach Erreichen der Reaktionstemperatur zusammen mit der (Meth)acrylsäure zu dem Gemisch gegeben.

Alternativ ist es möglich, den Katalysator und die (Meth)acrylsäure teilweise oder vollständig als Gemisch zusammen mit den anderen Verbindungen im Reaktionskessel vorzulegen. Ferner ist es möglich, den Katalysator oder die (Meth)acrylsäure zusammen mit den anderen Verbindungen im Reaktionskessel vorzulegen und die andere Komponente nach dem Erwärmen auf die Reaktionstemperatur zuzugeben.

Um eine vorzeitige unerwünschte Polymerisation der polymerisierbaren, Reaktionsprodukte und der gegebenenfalls zuzugebenden Reaktivverdünner vom Typ II während des gesamten Herstellungsprozesses und während der Lagerung des Reaktionsproduktes zu verhindern, empfiehlt es sich, bereits vor der Reaktion wenigstens 0,0005 bis 0,2 Gew.-%, bezogen auf das gesamte Reaktionsgemisch, einschließlich etwaiger Hilfs- und Zusatzmittel, an mindestens einem geeigneten Polymerisationsinhibitor zuzugeben. Der mindestens eine Polymerisationsinhibitor kann aber auch während oder nach der Umsetzung zugegeben werden. Der Polymerisationsinhibitor kann, falls erforderlich bis zu einer Menge von 2 Gew.-%, bevorzugt 0,01 bis 1 Gew.-%, bezogen auf das gesamte Reaktionsgemisch, zugegeben werden.

Als Polymerisationsinhibitoren sind die für radikalisch polymerisierbare Verbindungen üblicherweise verwendeten Polymerisationsinhibitoren geeignet, wie sie dem Fachmann bekannt sind.

Zur Stabilisierung gegen vorzeitige Polymerisation enthalten Harzmischungen und Reaktionsharzmörtel üblicherweise Polymerisationsinhibitoren, wie Hydrochinon, substituierte Hydrochinone, z.B. 4-Methoxyphenol, Phenothiazin, Benzochinon oder tert-Butylbrenzkatechin, wie beispielsweise in der EP 1935860 A1 oder EP 0965619 A1 beschrieben werden, stabile Nitroxylradikalen, auch N-Oxyl-Radikale genannt, wie Piperidinyl-N-oxyl oder Tetrahydropyrrol-N-oxyl, wie beispielsweise in der DE 19531649 A1 beschrieben. Besonders bevorzugt wird 4-Hydroxy-2,2,6,6-tetramethylpiperidin-N-oxyl (im Folgenden als Tempol bezeichnet) zur Stabilisierung verwendet, was den Vorteil hat, dass damit auch die Gelzeit eingestellt werden kann.

Bevorzugt sind die Polymerisationsinhibitoren unter phenolischen Verbindungen und nicht-phenolischen Verbindungen, wie stabilen Radikalen und/oder Phenothiazinen, ausgewählt.

Als phenolische Polymerisationsinhibitoren, die oft Bestandteil von kommerziellen radikalisch härtenden Reaktionsharzen sind, kommen Phenole, wie 2-Methoxyphenol, 4-Methoxyphenol, 2,6-Di-*tert*-butyl-4-methylphenol, 2,4-Di-*tert*-butylphenol, 2,6-Di-*tert-*butylphenol, 2,4,6-Trimethylphenol, 2,4,6-Tris(dimethylaminomethyl)phenol, 4,4'-Thio-bis(3-methyl-6-*tert*-butylphenol), 4,4'-Isopropylidendiphenol, 6,6'-Di-*tert-*butyl-4,4'-bis(2,6-di-*tert*-butylphenol), 1,3,5-Trimethyl-2,4,6-tris(3,5-di-*tert*-butyl-4-hydroxybenzyl)benzol, 2,2'-Methylen-di-*p*-cresol, Brenzkatechin und Butylbrenzkatechine, wie 4*-tert-*Butylbrenzkatechin, 4,6-Di-*tert*-butylbrenzkatechin, Hydrochinone, wie Hydrochinon, 2-Methylhydrochinon, 2-*tert*-Butylhydrochinon, 2,5-Di-*tert*-butylhydrochinon, 2,6-Di-*tert-*butylhydrochinon, 2,6-Dimethylhydrochinon, 2,3,5-Trimethylhydrochinon, Benzochinon, 2,3,5,6-Tetrachlor-1,4-benzochinon, Methylbenzochinon, 2,6-Dimethylbenzochinon, Naphthochinon, oder Gemische von zweien oder mehreren davon, in Frage.

Als nicht-phenolische Polymerisationsinhibitoren kommen vorzugsweise Phenothiazine, wie Phenothiazin und/oder Derivate oder Kombinationen davon, oder stabile organische Radikale, wie Galvinoxyl- und *N*-Oxyl-Radikale in Betracht.

Geeignete stabile *N*-Oxyl-Radikale (Nitroxylradikale) können unter 1-Oxyl-2,2,6,6-tetramethylpiperidin, 1-Oxyl-2,2,6,6-tetramethylpiperidin-4-ol (ebenso als TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet), Aluminium-N-nitrosophenylhydroxylamin, Diethylhydroxylamin ausgewählt werden, wie sie in der DE199 56 509 beschrieben sind. Ferner sind geeignete *N*-Oxylverbindungen Oxime, wie Acetaldoxim, Acetonoxim, Methylethylketoxim, Salicyloxim, Benzoxim, Glyoxime, Dimethylglyoxim, Aceton-*O*-(benzyloxycarbonyl)oxim und dergleichen. Ferner können in para-Position zur Hydroxylgruppe substituierte Pyrimidinol- oder Pyridinol-Verbindungen, wie sie in der nicht vorveröffentlichten Patentschrift DE 10 2011 077 248 B1 beschrieben sind, als Polymerisationsinhibitoren verwendet werden.

Die Polymerisationsinhibitoren können, abhängig von den gewünschten Eigenschaften und der Verwendung der Harzmischung, entweder alleine oder als Kombination von zweien oder mehreren davon verwendet werden. Die Kombination der phenolischen und der nicht-phenolischen Polymerisationsinhibitoren ermöglicht dabei einen synergistischen Effekt, wie auch die Einstellung einer im Wesentlichen driftfreien Einstellung der Gelierzeit der Reaktionsharzformulierung zeigt.

Zweckmäßig wird die Reaktion der Epoxidgruppen aufweisenden organischen Verbindung mit der (Meth)acrylsäure solange fortgesetzt, bis mindestens 80%, bevorzugt mindestens 90% und stärker bevorzugt mindestens 95% der Epoxidgruppen umgesetzt wurden.

Der Umsatz der Epoxidgruppen wird gemäß DIN 16945 während der Reaktion durch Titration der Epoxid-Gruppen fortlaufend ermittelt.

Die modifizierte Epoxy(meth)acrylatharze werden durch Veresterung eines Teils der β-Hydroxylgruppen des bei der Umsetzung der Epoxidgruppen aufweisenden organischen Verbindung mit (Meth)acrylsäure gebildeten Epoxy(meth)acrylats mit dem Anhydrid einer gesättigten C₃-C₅-Dicarbonsäure erhalten. Die gesättigte C₃-C₅-Dicarbonsäure ist unter Propandisäure (auch: Malonsäure), Butandisäure (auch: Bernsteinsäure) und Pentandisäure (auch: Glutarsäure) ausgewählt. Das Bernsteinsäureanhydrid ist erfindungsgemäß bevorzugt.

Die Erfinder haben herausgefunden, dass die Veresterung der β-Hydroxylgruppen nur mit den Anhydriden bei den gewählten Reaktionstemperaturen vollständig abläuft. Die bei der Veresterung gebildete freie Säuregruppe reagiert bei diesen Temperaturen nicht oder nur zu einem sehr geringen Teil, welcher vernachlässigbar ist, weiter. Im Falle der Dicarbonsäuren muss, um zum einen überhaupt eine Veresterung zu erreichen, die Reaktionstemperaturen höher gewählt werden. Diese sind dann allerdings so hoch, dass eine selektive Reaktion nur einer Säuregruppe der Dicarbonsäure nicht mehr gewährleistet ist und zumindest teilweise beide Säuregruppen reagieren und eine zu diesem Zeitpunkt unerwünschte Vernetzung bewirken.

Die Veresterung erfolgt ebenfalls bei etwa +80 bis +120°C, wobei das Anhydrid direkt zu dem Reaktionsgemisch der Umsetzung der Epoxidgruppen aufweisenden organischen Verbindung mit der (Meth)acrylsäure gegeben wird, ohne dass die entstehenden Produkte isoliert werden. Pro β-Hydroxylgruppe des bei der Umsetzung der Epoxidgruppen aufweisenden organischen Verbindung mit der (Meth)acrylsäure gebildeten Epoxy(meth)acrylats werden 1 bis 50 Mol% (Mol% / OH), bevorzugt 2 bis 30 Mol% und stärker bevorzugt 3 bis 15 Mol% (Mol% / OH) des Dicarbonsäureanhydrids eingesetzt.

Nach Zugabe des Dicarbonsäureanhydrids wird das Reaktionsgemisch für einen Zeitraum von sechs Stunden bei der Reaktionstemperatur von +80°C bis +120°C gehalten. Nach beendeter Umsetzung wird das Reaktionsgemisch auf Raumtemperatur abgekühlt.

Die wie oben beschrieben hergestellten Reaktionsprodukte können ohne den Zusatz von Lösungsmitteln zur Anwendung gelangen. Gegebenenfalls können sie auch mit Reaktivverdünnern vom Typ II verdünnt werden, um die gewünschte Viskosität einzustellen.

Geeignete Reaktivverdünner vom Typ II sind in den Anmeldungen EP 1 935 860 A1 und DE 195 31 649 A1 beschrieben. Vorzugsweise enthält die Harzmischung als Reaktivverdünner einen (Meth)acrylsäureester, wobei besonders bevorzugt aliphatische oder aromatische C₅-C₁₅-(Meth)acrylate ausgewählt werden. Geeignete Beispiele umfassen: Hydroxypropyl(meth)acrylat, 1,2-Ethandioldi(meth)acrylat, 1,3-Propandioldi(meth)acrylat, 1,3-Butandioldi(meth)acrylat, 1,4-Butandioldi(meth)acrylat, Trimethylolpropantri(meth)acrylat, Phenethyl(meth)acrylat, Tetrahydrofurfuryl(meth)acrylat, Ethyltriglykol(meth)acrylat, *N,N*-Dimethylaminoethyl(meth)acrylat, *N,N-*Dimethylaminomethyl(meth)acrylat, Acetoacetoxyethyl(meth)acrylat, Isobornyl(meth)acrylat, 2-Ethylhexyl(meth)acrylat, Diethylenglykoldi(meth)acrylat, Methoxypolyethylenglykolmono(meth)acrylat, Trimethylcyclohexyl(meth)acrylat, 2-Hydroxyethyl(meth)acrylat, Dicyclopentenyloxyethyl(meth)acrylat und/oder Tricyclopentadienyldi(meth)acrylat, Bisphenol-A-(meth)acrylat, Novolakepoxidi(meth)acrylat, Di-[(meth)acryloyl-maleoyl]-tricyclo-[5.2.1.0.^{2.6}]-decan, Dicyclopentenyloxyethylcrotonat, 3-(Meth)acryloyl-oxymethyl-tricylo-[5.2.1.0.^{2.6}]-decan, 3-(Meth)cyclopentadienyl(meth)acrylat, Isobornyl(meth)acrylat und Decalyl-2-(meth)acrylat; PEG-di(meth)acrylate, wie PEG200-di(meth)acrylat, Tetraethylenglykoldi(meth)acrylat, Solketal(meth)acrylat, Cyclohexyl(meth)acrylat, Phenoxyethyldi(meth)acrylat, Methoxyethyl(meth)acrylat, Tetrahydrofurfuryl(meth)acrylat, *tert*-Butyl(meth)acrylat und Norbornyl(meth)acrylat. Grundsätzlich können auch andere übliche radikalisch polymerisierbaren Verbindungen, allein oder im Gemisch mit den (Meth)acrylsäureestern, eingesetzt werden, z. B. Styrol, α-Methylstyrol, alkylierte Styrole, wie *tert*-Butylstyrol, Divinylbenzol und Allylverbindungen, wobei die nicht kennzeichnungspflichtigen Vertreter davon bevorzugt sind.

Die wie oben beschrieben, hergestellten Produkte stellen wertvolle, mittels geeigneter Radikale liefernden Substanzen wie (Hydro)Peroxiden, gegebenenfalls in Gegenwart von Beschleunigern, aushärtbare Systeme dar.

Bevorzugt werden die wie oben beschrieben, hergestellten Produkte als Bindemittelkomponenten für Klebe-, Haft-, Dicht- und Beschichtungsmittel eingesetzt. Besonders bevorzugt werden die erfindungsgemäß hergestellten Produkte als Bindemittel für radikalisch härtbare, insbesondere kalthärtende Mörtelmassen zur chemischen Befestigung verwendet.

Reaktionsharzmörtel werden in der Regel herstellt, indem die zur Herstellung des Basisharzes erforderlichen Ausgangsverbindungen gegebenenfalls zusammen mit Katalysatoren und Lösungsmitteln, insbesondere Reaktivverdünner, in einem Reaktor gegeben und miteinander zur Reaktion gebracht werden. Nach Beendigung der Reaktion und gegebenenfalls bereits zu Beginn der Reaktion werden zu dem Reaktionsgemisch Polymerisationsinhibitoren für die Lagerstabilität gegeben, wodurch der sogenannte Harz-Masterbatch erhalten wird. Zu dem Harz-Masterbatch werden häufig Beschleuniger für die Härtung des Basisharzes, gegebenenfalls weitere Polymerisationsinhibitoren, die gleich oder ungleich dem Polymerisationsinhibitor für die Lagerstabilität sein können, zur Einstellung der Gelzeit, und gegebenenfalls weiteres Lösungsmittel, insbesondere Reaktivverdünner, gegeben, wodurch die Harzmischung erhalten wird. Zur Einstellung der Gelzeit und der Reaktivität können weitere 0,005 bis 3 Gew.-%, vorzugsweise 0,05 bis 1 Gew.-%, bezogen auf die Harzmischung, eines Polymerisationsinhibitors enthalten sein. Diese Harzmischung wird zur Einstellung verschiedener Eigenschaften, wie der Rheologie und der Konzentration des Basisharzes, mit anorganischen und/oder organischen Zuschlagstoffen versetzt, wodurch der Reaktionsharzmörtel erhalten wird.

Eine bevorzugte Harzmischung enthält dementsprechend mindestens ein Basisharz, mindestens einen Reaktivverdünner, mindestens einen Beschleuniger, mindestens einen Polymerisationsinhibitoren. Ein Reaktionsharzmörtel enthält neben der eben beschriebenen Harzmischung anorganische und/oder organische Zuschlagstoffe, wobei anorganische Zuschlagstoffe besonders bevorzugt sind.

Die Herstellung der Epoxy(meth)acrylatharze zur erfindungsgemäßen Verwendung soll in den nachfolgenden Beispielen näher erläutert werden, ohne darauf beschränkt zu werden.

### AUSFÜHRUNGSBEISPIELE

### A) Harz-Masterbatch Synthesen

### Beispiel 1 (monomeres Harz, n ∼ 0,1)

223 g Bisphenol-A-diglycidylether (EEW (DIN 16945) von 182-192 g/Val, Epilox ® A 19-03; LEUNA-Harze GmbH), wird bei Raumtemperatur vollständig im Reaktor vorgelegt und mit 110 g Methacrylsäure, 0,1 g Phenothiazin sowie 2 g Tetraethylammoniumbromid versetzt. Die Reaktionsmischung wird über 30 Minuten linear auf ca. 80°C erwärmt und für 20 Stunden bei dieser Temperatur gehalten.

Der Umsatz der Epoxidgruppen wird während der Reaktion durch Titration der Epoxid-Gruppen gemäß DIN 16945 fortlaufend ermittelt.

Sobald ein Umsatz von mindestens 97% erreicht wird, werden 20 Mol% / OH Bernsteinsäureanhydrid zugegeben und weiter bei einer Temperatur von 80°C gerührt. Nach einer Reaktionszeit von 6 Stunden wird das Reaktionsgemisch auf Raumtemperatur abgekühlt. Man erhält so einen gebrauchsfertigen Harz-Masterbatch.

### Beispiel 2 (polymeres Harz, n ∼ 1)

273 g Bisphenol-A-diglycidylether (EEW (DIN 16945) 300-340 g/Val, Epilox ® A 32-02; LEUNA-Harze GmbH) werden bei Raumtemperatur vollständig im Reaktor vorgelegt und mit 88 g PEG200-dimethacrylat, 79 g Methacrylsäure, 0,1 g Phenothiazin sowie 3 g Tetraethylammoniumbromid versetzt. Die Reaktionsmischung wird über 30 Minuten linear auf ca. 80°C erwärmt und für 20 Stunden bei dieser Temperatur gehalten.

Der Umsatz der Epoxidgruppen wird während der Reaktion durch Titration der Epoxid-Gruppen gemäß DIN 16945 fortlaufend ermittelt.

Sobald ein Umsatz von mindestens 97% erreicht wird, werden 10 Mol% / OH Bernsteinsäureanhydrid zugegeben und bei einer Temperatur von 80°C gerührt. Nach einer Reaktionszeit von 6 Stunden wird das Reaktionsgemisch auf Raumtemperatur abgekühlt. Man erhält so einen gebrauchsfertigen Harz-Masterbatch.

### Beispiel 3 (polymeres Harz, n ∼ 2)

324 g Bisphenol-A-diglycidylether (EEW (DIN 16945) 450-500 g/val, Epilox ® A 50-02; LEUNA-Harze GmbH) werden bei Raumtemperatur vollständig im Reaktor vorgelegt und mit 97 g PEG200-dimethacrylat, 63 g Methacrylsäure, 0,04 g Phenothiazin, 0,08g 4-Hydroxy-2,2,6,6-tetramethylpiperidinyl-N-oxyl sowie 3 g Tetraethylammoniumbromid versetzt. Die Reaktionsmischung wird über 30 Minuten linear auf ca. 100°C erwärmt und für 5 Stunden auf dieser Temperatur gehalten.

Der Umsatz der Epoxidgruppen wird während der Reaktion durch Titration der Epoxid-Gruppen gemäß DIN 16945 fortlaufend ermittelt.

Sobald ein Umsatz von mindestens 97% erreicht wird, werden 10 Mol% / OH Bernsteinsäureanhydrid zugegeben und bei einer Temperatur von 100°C gerührt. Nach einer Reaktionszeit von 2 Stunden wird das Reaktionsgemisch auf Raumtemperatur abgekühlt. Man erhält so einen gebrauchsfertigen Harz-Masterbatch.

### B) Harzmischungen

Zur Herstellung der Harzmischungen werden die wie oben beschrieben hergestellten Harz-Masterbatches A bis C jeweils mit PEG200DMA, 1,4-Butandioldimethacrylat (BDDMA), *tert*-Butylbrenzkatechin (tBBK) und 4-Hydroxy-2,2,6,6-tetramethylpiperidin-1-oxyl (Tempol) gemischt. Die verwendeten Mengen sind in der nachfolgenden Tabelle 1 aufgelistet. Anschließend wird die Gelzeit der jeweils resultierenden Harzmischung mit einem aromatischen Amin auf ca. 6 Minuten eingestellt

**Tabelle 1: Mengen der Komponenten zur Herstellung der Harzmischungen**

| **Komponente** | **A1 / B1 / C1 (jeweils n ∼ 0,1)** | **A2 / B2 (jeweils n ∼ 1)** | **A3 / B3 / C3 (jeweils n ∼ 2)** |
|---|---|---|---|
| | **Menge [Gew.-%]** | | |
| Harz-Masterbatch | 39,2 | 42,54 | 36,14 |
| PEG200DMA | 25,4 | 19,6 | 23,5 |
| BDDMA | 35,3 | 37,8 | 40,3 |
| tBBK | 0,05 | 0,04 | 0,04 |
| Tempol | 0,015 | 0,015 | 0,015 |

Die Bestimmung der Gelzeit erfolgt mit einer handelsüblichen Vorrichtung (GELNORM®-Gel Timer) bei einer Temperatur von 25°C. Hierzu werden jeweils die A und die B Komponente im Volumenverhältnis 3:1 gemischt und unmittelbar nach dem Mischen im Silikonbad auf 25°C temperiert und die Temperatur der Probe gemessen. Die Probe selbst befindet sich dabei in einem Reagenzglas, das in einen im Silikonbad versenkten Luftmantel zur Temperierung gesetzt wird.

Die Wärmeentwicklung der Probe wird gegen die Zeit aufgetragen. Die Auswertung erfolgt gemäß DIN16945, Blatt 1 und DIN 16916. Die Gelzeit ist die Zeit, bei der ein Temperaturanstieg um 10K erreicht wird, hier von 25°C nach 35°C.

Man erhält so eine gebrauchsfertige lagerstabile Harzmischung.

### C) Reaktionsharzmörtel

Zur Herstellung der Hybridmörtel werden die Harzmischungen mit 30-45 Gewichtsanteilen Quarzsand, 15-25 Gewichtsanteilen Zement und 1-5 Gewichtsanteilen pyrogener Kieselsäure im Dissolver zu einer homogenen Mörtelmasse gemischt.

### D) Härterkomponente

Zur Herstellung der Härterkomponente werden 13 g Dibenzoylperoxid, 23 g Wasser, 1 g pyrogene Kieselsäure, 19 g Aluminiumoxid und 46 g Quarzmehl geeigneter Korngrößenverteilung im Dissolver zu einer homogenen Masse gemischt.

### Bestimmung der Versagensverbundspannungen (τ)

Zur Bestimmung der Versagensverbundspannung der ausgehärteten Masse verwendet man Ankergewindestangen M12, die in Bohrlöcher in Beton mit einem Durchmesser von 14 mm und einer Bohrlochtiefe von 72 mm mit den Reaktionsharzmörtel-Zusammensetzungen der Beispiele und Vergleichsbeispiele eingedübelt werden. Man ermittelt die mittleren Versagenslasten durch zentrisches Ausziehen der Ankergewindestangen. Es werden jeweils drei Ankergewindestangen eingedübelt und nach 24h Aushärtung ihre Lastwerte bestimmt. Die hierbei ermittelten Verbundtraglasten τ (N/mm²) sind als Mittelwert in der nachfolgenden Tabelle 2 aufgeführt.

Verschiedene Bohrlochbedingungen und/oder Aushärtebedingungen wurden wie nachfolgend aufgelistet getestet.

| Testbedingung | Anmerkung |
|---|---|
| Referenz | gut gereinigtes, hammergebohrtes Bohrloch, Aushärtung bei Raumtemperatur (+20°C) |
| -10°C | Referenzlöcher, Setzen und Aushärten bei einer Untergrundtemperatur von -10°C |
| +40°C | Referenzlöcher, Setzen und Aushärten bei einer Untergrundtemperatur von +40°C |

**Tabelle 2: Verbundtraglasten (τ) der modifizierten BisGMA-Harze**

| **Beispiele** | | **1** | **2** | **3** |
|---|---|---|---|---|
| | | **(n∼0,1)** | **(n∼1)** | **(n∼2)** |
| Verbundtraglasten τ [N/mm²] | Ref. | 21,5±0,5 | 20,0±1,6 | 18,0±2,0 |
| | -10°C | 18,0±1,6 | 16,8±1,3 | 13,6±1,2 |
| | +40°C | 21,4±1,2 | 20,7±1,6 | 19,7±0,9 |

## Patentansprüche

1. Verwendung eines modifizierten Epoxy(meth)acrylates erhältlich durch ein Verfahren, bei dem Epoxidgruppen aufweisende organische Verbindungen mit (Meth)acrylsäure in Gegenwart eines geeigneten Katalysators umgesetzt werden und nachdem mindestens 80% der Epoxidgruppen umgesetzt wurden, das Produkt teilweise mit dem Anhydrid einer gesättigten Dicarbonsäure umgesetzt wird, als Bindemittel in radikalisch härtbaren Harzmischungen oder in radikalisch härtbaren Reaktionsharzmörtel-Zusammensetzungen.

2. Verwendung nach Anspruch 1, **dadurch gekennzeichnet, dass** die Epoxidgruppen aufweisende organische Verbindungen ein Zahlenmittel der Molmasse *M̅ₙ* im Bereich von 129 bis 2.400 g/Mol aufweisen.

3. Verwendung nach Anspruch 2, **dadurch gekennzeichnet, dass** die Epoxidgruppen aufweisenden organischen Verbindungen eine Epoxidäquivalentmasse (EEW) im Bereich von 87 bis 1.600 g/Val aufweisen.

4. Verwendung nach einem der vorhergehenden Ansprüche, **dadurch gekennzeichnet, dass** pro Epoxidäquivalent 0,7 bis 1,2 Carboxyäquivalente (Meth)acrylsäure eingesetzt werden.

5. Verwendung nach einem der vorhergehenden Ansprüche, **dadurch gekennzeichnet, dass** pro β-Hydroxylgruppe des bei der Umsetzung der Epoxidgruppen aufweisenden organischen Verbindung mit der (Meth)acrylsäure gebildeten Epoxy(meth)acrylats 1 bis 50 Mol% des Anhydrids einer gesättigten Dicarbonsäure eingesetzt werden.

6. Verwendung nach einem der vorhergehenden Ansprüche, **dadurch gekennzeichnet, dass** die Dicarbonsäure eine C₃-C₅-Dicarbonsäure ist.

7. Verwendung nach einem der vorhergehenden Ansprüche, **dadurch gekennzeichnet, dass** die Umsetzung der Epoxidgruppen aufweisenden organischen Verbindung mit der (Meth)acrylsäure in Gegenwart eines co-polymerisierbaren Monomers durchgeführt wird.

8. Verwendung nach einem der vorhergehenden Ansprüche, **dadurch gekennzeichnet, dass** die Umsetzung der Epoxidgruppen aufweisenden organischen Verbindung mit der (Meth)acrylsäure in Gegenwart eines Polymerisationsinhibitors durchgeführt wird.

## Claims

1. Use of a modified epoxy(meth)acrylate that may be obtained through a process, in which organic compounds that have epoxide groups are converted with (meth)acrylic acid in the presence of a suitable catalyst and then after at least 80% of the epoxide groups were converted, the product is partly converted with the anhydride of a saturated dicarboxylic acid, as a binder in radically hardenable resin mixtures or in radically hardenable reaction resin mortar compounds.

2. Use according to claim 1, **characterised in that** the organic compounds that have epoxide groups have an average number of the molar mass *Mₙ* in the range from 129 to 2,400 g/Mol.

3. Use according to claim 2, **characterised in that** the organic compounds that have epoxide groups have an epoxide equivalent mass (EEW) in the range from 87 to 1,600 g/Val.

4. Use according to one of the previous claims, **characterised in that** 0.7 to 1.2 carboxyl equivalents of (meth)acrylic acid are used per epoxide equivalent.

5. Use according to one of the previous claims, **characterised in that** 1 to 50 mole % of the anhydride of a saturated dicarboxylic acid are used per ß-hydroxyl group of the epoxy(meth)acrylate formed in the conversion of the organic compound that has epoxide groups with the (meth)acrylic acid.

6. Use according to one of the previous claims, **characterised in that** the dicarboxylic acid is a C₃-C₅-dicarboxylic acid.

7. Use according to one of the previous claims, **characterised in that** the conversion of the organic compound that has epoxide groups with the (meth)acrylic acid is carried out in the presence of a co-polymerisable monomer.

8. Use according to one of the previous claims, **characterised in that** the conversion of the organic compound that has epoxide groups with the (meth)acrylic acid is carried out in the presence of a polymerisation inhibitor.

## Revendications

1. Utilisation d'un époxy(méth)acrylate modifié, pouvant être obtenu par un procédé dans lequel des composés organiques présentant des groupes époxydes sont mis à réagir avec de l'acide (méth)acrylique en présence d'un catalyseur approprié, et, après qu'au moins 80 % des groupes époxydes ont réagi, le produit est partiellement mis à réagir avec l'anhydride d'un acide dicarboxylique saturé, servant de liant dans des mélanges de résines à durcissement radicalaire ou des compositions de mortier à base de résine de réaction à durcissement radicalaire.

2. Utilisation selon la revendication 1, **caractérisée en ce que** les composés organiques présentant des groupes époxydes présentent une masse moléculaire moyenne en nombre *M̅ₙ* comprise dans la plage de 129 à 2400 g/mol.

3. Utilisation selon la revendication 2, **caractérisée en ce que** les composés organiques présentant des groupes époxydes présentent une masse équivalente d'époxyde (EEW) comprise dans la plage de 87 à 1600 g/val.

4. Utilisation selon l'une des revendications précédentes, **caractérisée en ce qu'**on utilise par équivalent époxyde 0,7 à 1,2 équivalents carboxy d'acide (méth)acrylique.

5. Utilisation selon l'une des revendications précédentes, **caractérisée en ce qu'**on utilise par groupe β-hydroxyle de l'époxy(méth)acrylate formé lors de la réaction du composé organique présentant des groupes époxydes avec l'acide (méth)acrylique 1 à 50 % en moles de l'anhydride d'un acide dicarboxylique saturé.

6. Utilisation selon l'une des revendications précédentes, **caractérisée en ce que** l'acide dicarboxylique est un acide dicarboxylique en C₃-C₅.

7. Utilisation selon l'une des revendications précédentes, **caractérisée en ce que** la réaction du composé organique présentant des groupes époxydes avec l'acide (méth)acrylique est mise en oeuvre en présence d'un monomère copolymérisable.

8. Utilisation selon l'une des revendications précédentes, **caractérisée en ce que** la réaction du composé organique présentant des groupes époxydes avec l'acide (méth)acrylique est mise en oeuvre en présence d'un inhibiteur de polymérisation.
